(19) Europäisches Patentamt
European Patent Office
Office européen des brevets

(11) Veröffentlichungsnummer: **0 421 915 A1**

# (12) EUROPÄISCHE PATENTANMELDUNG

(21) Anmeldenummer: **90730011.5**

(51) Int. Cl.5: **A61B 5/20**, G01F 1/52

(22) Anmeldetag: **28.09.90**

(30) Priorität: **30.09.89 DE 3933025**

(43) Veröffentlichungstag der Anmeldung:
**10.04.91 Patentblatt 91/15**

(84) Benannte Vertragsstaaten:
**AT BE CH DE DK ES FR GB GR IT LI LU NL SE**

(71) Anmelder: **Wiest, Peter P., Dipl.-Ing.**
**Hessenallee 8**
**W-1000 Berlin 19(DE)**

(72) Erfinder: **Wiest, Peter P., Dipl.-Ing.**
**Hessenallee 8**
**W-1000 Berlin 19(DE)**

(74) Vertreter: **Lüke, Dierck-Wilm, Dipl.-Ing.**
**Gelfertstrasse 56**
**W-1000 Berlin 33(DE)**

## (54) Einrichtung zur Messung der Harnströmung (Uroflow) eines Patienten.

(57) Eine Einrichtung zur Messung der Harnströmung ( Uroflow) eines Patienten, besteht aus einem mit einem Trichter (2) und einem Ausflußstutzen (30) versehenen Meßkopf (1) und aus einer elektronischen Auswerteeinrichtung (9).

Zur Vereinfachung des mechanischen Aufbaus und zur Erhöhung der Meßgenauigkeit ist vorgesehen, daß der Meßkopf 1 mit einem den Ausflußstutzen 30 bildenden geschlitzten Staurohr 4 und mit einem den Staudruck messenden Druckaufnehmer 18 versehen ist und daß die elektronische Auswerteeinrichtung 9 die Durchflusswerte aus dem gemessenen Staudruck errechnet.

EP 0 421 915 A1

# EINRICHTUNG ZUR MESSUNG DER HARNSTRÖMUNG (UROFLOW) EINES PATIENTEN

Die Erfindung bezieht sich auf eine Einrichtung zur Messung der Harnströmung (Uroflow) eines Patienten, aus einem mit einem Trichter und einem Ausflußstutzen versehenen Meßkopf und aus einer elektronischen Auswerte-Einrichtung.

Bekannte Meßeinrichtungen dieser Art messen als physikalische Größe entweder das Volumen des Urins oder den Harnfluß. Dies erfolgt rotationsdynamisch, gravimetrisch, kapazitiv oder speziell bei der Volumenmessung über den Druck der Flüssigkeitshöhe in einem Auffangbecher.

Eine Meßvorrichtung der gattungsgemäßen Art ist aus der DE-PS 30 07 855 vorbekannt. Hierbei ist der Meßkopf aus einem innerhalb eines Gehäuses frei schwingbar gelagerten, vom Urin des Patienten durchflossenen, mit leichtem Gefälle im wesentlichen horizontal angeordneten U-förmigen Meßrohr gebildet, in dessen Einlaufstutzen der Trichter mündet und dessen Auslaufstutzen ausserhalb des Gehäuses angeordnet ist. Der Urin des Patienten durchströmt das U-förmige Meßrohr, in welchem sich nur eine Teilmenge der Urinmenge einer vollen Miktion befindet, so daß das Meßrohr sehr klein ausgebildet werden kann. Nachteilig hierbei ist jedoch die Notwendigkeit der schwingbaren Lagerung des U-förmigen Meßrohres innerhalb des Gehäuses.

Eine Uroflow-Meßeinrichtung der gattungsgemäßen Art muß in der urologischen Praxis in der Toilette installiert werden können, so daß der Urin dort direkt abfließen kann. Dies läßt nur eine Einrichtung zu, die den Urin nicht sammelt. Bei klinischen, urodynamischen Untersuchungen wird jedoch auf die genaue Volumenmessung wesentlich mehr Wert gelegt. Der Urin wird in einem Becher an der Meßeinrichtung gesammelt. Ferner ist die exakte zeitliche Darstellung der Flowkurve sehr wichtig für die Diagnose. Dies ist nur möglich, wenn der Flow direkt gemessen wird. Wenn die auf dem Volumen basierende Messung auf die doppelte Differenzierung angewiesen ist, um den Flowanstieg darzustellen, wird das Rechenergebnis auf Grund von Störeinflüssen viel zu ungenau, wie z.B. Erschütterungen und Wellenbewegungen der gesammelten Flüssigkeit. In der urologischen Praxis ist das Entleeren der gesammelten Flüssigkeit bei der großen Anzahl der Uroflow-Tests als Voruntersuchung zu aufwendig.

Bei den den Flow messenden Einrichtungen gibt es gerade im minimalen Flowbereich große Fehler bei der Volumen-Berechnung durch Integration über die Zeit. In dieser Hinsicht ist die Volumenmessung der Flowmessung überlegen.

Der Erfindung liegt die Aufgabe zugrunde, eine Meßeinrichtung für die Harnströmung (Uroflow) eines Patienten zu schaffen, welche die Vorteile der Flowmessung mit den Vorteilen der Volumenmessung verbindet, wobei die Einrichtung auch ohne Sammlung des Urins in der urologischen Praxis eingesetzt werden können soll und wobei die Meßgenauigkeit von Flow und Volumen für die klinischen, urodynamischen Untersuchungen ausreichend sein soll.

Zur Lösung dieser Aufgabe sieht die Erfindung vor, daß der Meßkopf mit einem den Ausflußstutzen bildenden geschlitzten Staurohr und mit einem den Staudruck messenden Druckaufnehmer versehen ist und daß die Auswerte-Einrichtung die Flowwerte aus dem gemessenen flowproportionalen Staudruck errechnet. Hierdurch wird ermöglicht, daß sowohl der Flow als auch das Volumen ohne bewegliche Bauteile gemessen werden können, wobei die Meßgenauigkeit von Flow und Volumen sehr hoch ist.

In einer bevorzugten Ausführungsform weist der Meßkopf einen mit dem Trichter verbundenen vertikalen Zulaufraum, einen vom geschlitzten Staurohr durchdrungenen Abführraum, einen mit dem Druckaufnehmer verbundenen vertikalen Druckmeßraum und einen den Zulaufraum, den Abführraum und den Druckmeßraum verbindenden horizontalen Verteilraum auf, wobei die Unterkanten des Vertikalschlitzes im Staurohr, des Zulaufraumes und des Druckraumes in einer gemeinsamen horizontalen Ebene angeordnet sind, welche die obere Ebene des horizontalen Verteilraumes bildet. Hierbei bildet sich eine Stauhöhe des Urins im Zulaufraum aus, welcher über den horizontalen Verteilraum sowohl mit dem vom geschlitzten Staurohr durchdrungenen Abführraum als auch mit dem vertikalen Druckmeßraum verbunden ist. Die Flüssigkeitssäule im Zulaufraum sperrt den vertikalen Druckmeßraum ab, in dem sich eine Luftsäule ausbildet, welche den Staudruck auf den Druckaufnehmer überträgt.

In weiterer vorteilhafter Ausgestaltung der Erfindung ist der Verteilraum durch eine am Meßkopf lösbare und flüssigkeitsdicht angeflanschte Schale gebildet, in welche das geschlitzte Staurohr eingesetzt ist, das einen vertikalen Schlitz geringer Breite aufweist .

Um sowohl den Flow als auch das Volumen als dem Meßwert proportionale Druckgrößen mit zwei gleichen Drucksensoren messen zu können, sieht die Erfindung ferner vor, daß der Meßkopf mit dem Ausflußstutzen auf einem Auffangbecher aufgesetzt ist und daß mit einem weiteren, im Meßkopf angeordneten Druckaufnehmer das Gesamtvolumen des Urins durch Messung der Füllhöhe des Auffangbehälters über den statischen Druck des im Auffangbehälter gesammelten Urins gemessen wird. Der zweite Druckaufnehmer mißt dabei die Füllhöhe des Urins im Auffangbehälter über ein Meßrohr, das an den Meßkopf angeflanscht ist, bis zum Boden des Auffangbehälters reicht und an dessem oberen Ende der zweite Meßwertaufnehmer angesetzt ist.

Schließlich sind der Meßkopf und die beiden Druckaufnehmer in getrennten Baueinheiten untergebracht, welche voneinander leicht trennbar sind. Auf diese Weise können alle mit dem Urin in Berührung kommenden Teile, insbesondere der Meßkopf selbst mit der Auffangschale, dem geschlitzten Staurohr und dem Meßrohr sowie der Auffangbehälter aus Kunststoff gebildet sein, die einfach gereinigt und desinfiziert werden können sowie als Ersatzteile kostengünstigherstellbar sind, wohingegen die Druckaufnehmer in einer separaten Baueinheit untergebracht sind.

Die Erfindung ist nachfolgend anhand eines in der Zeichnung dargestellten Ausführungsbeispieles einer Meßeinrichtung für die Harnströmung (Uroflow) eines Patienten näher erläutert.

Die einzige Figur zeigt einen Vertikal schnitt durch die Meßeinrichtung.

Die Meßeinrichtung besteht aus einem Meßkopf 1, einem Trichter 2, einer am Meßkopf 1 angeflanschten Auffangschale 3, einem Schlitzrohr 4, einem Meßrohr 5, einem Auffangbehälter 6 mit Deckel 7, einer Druckaufnehmereinheit 8 sowie einer elektronischen Auswerte-Einrichtung 9.

Der Meßkopf 1, der insbesondere aus Kunststoff besteht, trägt auf seiner Oberseite den Trichter 2, der in eine Aufnahmeöffnung 11 des Meßkopfes 1 eingesetzt ist. Diese mündet in einen vertikalen Zulaufraum 12, der am unteren Ende offen ist. Parallel zum Zulaufraum 12 erstreckt sich in vertikaler Richtung innerhalb des Meßkopfes 1 ein Abführraum 13, in den das geschlitzte Staurohr 4 eingesetzt ist, das einen vertikalen Schlitz 14 der Breite S aufweist. Das ebenfalls aus Kunststoff bestehende geschlitzte Staurohr 4 ist in der aus Kunststoff bestehenden Auffangschale 3 fest eingesetzt, welche unter Zwischenschaltung einer Dichtung 15 flüssigkeitsdicht mit der Unterseite des Meßkopfes 1 verbunden ist.

Parallel zum Zulaufraum 12 ist auf der dem Abführraum 13 gegenüberliegenden Seite des Meßkopfes 1 ein vertikaler Druckmeßraum 16 ausgebildet, der nach unten offen ist und oben über eine Anschlußbohrung 17 mit der Druckaufnehmereinheit 8 verbunden ist. Dem Druckmeßraum 16 ist in der Druckaufnehmereinheit 8 ein erster Drukkaufnehmer 18 zugeordnet, der über eine elektrische Anschlußleitung 19 mit der Auswerte-Einrichtung 9 verbunden ist.

Die unterhalb des Meßkopfes 1 angeflanschte Auffangschale 3 bildet einen horizontalen Verteilraum 10, wobei die Unterkanten des vertikalen Schlitzes14 im Staurohr 4, des Zulaufraumes 12 und des Druckraumes 16 in einer gemeinsamen horizontalen Ebene 20 liegen , welche die obere Ebene des horizontalen Verteilraumes 10 bildet.

Der Meßkopf 1 trägt ferner das zum Staurohr 4 parallele, jedoch wesentlich längere Meßrohr 5, das über eine Bohrung 21 mit einem zweiten Druckaufnehmer 22 in der Druckaufnehmereinheit 8 verbunden ist. Dieser zweite Druckaufnehmer 22 ist über eine elektrische Steuerleitung 23 ebenfalls mit der Auswerte-Einrichtung 9 elektrisch verbunden. Die Druckaufnehmereinheit 8 ist ein gegenüber dem Meßkopf 1 separates Bauteil, dessen Meßbohrungen 24, 25 mit den Meßbohrungen 17, 21 der Druckmeßkammer 16 bzw. des Meßrohres 5 druckdicht verbunden sind. Hierzu sind im Übergangsbereich O-Ringe 26 eingesetzt.

Der Meßkopf 1 ist mit seiner die Unterseite verschließenden Auffangschale 3 auf den Deckel 7 des Auffangbehälters 6 aufgesetzt, wobei sowohl das Staurohr 4 als auch das Meßrohr 5 den Deckel 7 abgedichtet durchdringen. Der Deckel 7 ist über federbelastete T-Bolzen 27 fest auf den Auffangbehälter 6 aufgeklemmt. Das Meßrohr 5 reicht tief bis in den Auffangbehälter 6 hinein, wobei die untere Öffnung des Meßrohres 5 dicht über dem Boden 28 des Auffangbehälters 6 angeordnet ist.

Die beschriebene Meßvorrichtung arbeitet wie folgt:

Der Druckaufnehmer 22, der mit dem Meßrohr 5 verbunden ist, mißt über die Luftsäule im Meßrohr 5 die Füllhöhe H des Urins 29 im Auffangbehälter 6.

Der Druckaufnehmer 18 mißt die Stauhöbe h im Zulaufraum 12 des Meßkopfes 1 über die Luftsäule in dem Druckmeßraum 16. Die augenblickliche Stauhöhe h wird durch den Urinzufluß über den Trichter 2 und den Zulaufraum 12 über das Abfließverhalten des Urins über das geschlitzte Staurohr 4 mit dem vertikalen Schlitz 14 bestimmt. Der Zufluß und der Abfluß halten sich in der jeweiligen Stauhöhe h das Gleichgewicht. Unter Vernachlässigung von Reibung und Oberflächenspannung gelten folgende Beziehungen:

(1) $C_o = \sqrt{2 \times g \times h'}$ (Gleichung nach Bernoulli)

$$(2)\quad \mathring{V} = S \times \int_0^h C_o \times dh$$

$C_o$ = Ausflußgeschwindigkeit aus dem Schlitz 14

g = Erdbeschleunigung (9,81 m/sec$^2$)

h' = Stauhöhe über dem Auflußstrahl

$\mathring{V}$ = Flow durch das geschlitzte Staurohr (Volumen/sec.) (ml/sec.)

S = Schlitzbreite
h = Stauhöhe

Nach Gleichung (2) wird von der elektronischen Auswerte-Einrichtung 9 der Flow aus der gemessenen Stauhöhe h ermittelt.

Bei Benutzung des Auffangbehälters 6 kann die elektronische Auswerte-Einrichtung 9 prüfen, ob anhand der Volumenmessung die Flowkurve die richtige Höhe hat und kann diese entsprechend korrigieren. Dies ist während der Messung nach jeder größeren Flowänderung oder nach dieser möglich, wenn die Flow- und Volumenwerte in der elektronischen Auswerte-Einrichtung 9 abgespeichert sind.

Bei geringen Anforderungen an die Genauigkeit kann auf den Auffangbehälter 6 verzichtet werden. Der Meßkopf 1 wird dann einfach in einer Toilette installiert. Der Auffangbehälter 6 dient dann nur der Kalibration des Flow-Meßteiles im Meßkopf 1 mit dem Druckaufnehmer 18. Dazu wird ein definiertes Volumen an Wasser mit unterschiedlichen Flowgeschwindigkeiten durch den Trichter 2 gegossen. Nach jeder Flowänderung wird der mittlere, vorherige Flow mit der vorherigen Volumenänderung verglichen,und für diesen Flowbereich wird der Korrekturwert in einem nichtflüchtigen Speicher der elektronischen Auswerte-Einrichtung 9 abgelegt.

Besonders vorteilhaft ist die Trennbarkeit der Druckaufnehmereinheit 8 mit den beiden Druckaufnehmern 18 und 22 vom Meßkopf 1. Die den Verteilungsraum 10 bildende Auffangschale 3 mit dem geschlitzten Staurohr 4 läßt sich ebenfalls einfach vom Meßkopf 1 trennen. Dies ermöglicht eine einfache Reinigung und Desinfektion der vom Urin berührten Bauteile, während die Druckaufnehmer 18, 22 bei diesen Arbeiten nicht chemisch oder mechanisch gefährdet werden. Die vom Urin berührten Bauteile können auch als preiswerte Einwegartikel aus Kunststoff ausgebildet sein, welche nach einer kurzen Gebrauchszeit, z.B. 1 Woche, erneuert werden.

Der Auffangbehälter 6 kann durch eine Drehung um seine Achse am Meßkopf 1 spielfrei eingerastet werden, welcher wiederum an einem Stativausleger montierbar ist.

Durch Verwendung von baugleichen Druckaufnehmern 18, 22 lassen sich diese von einem Leiterpaar mit Energie versorgen. Als elektronische Auswerte-Einheit 8 dient eine Computerschaltung, welche über einen Drucker sowohl die Flowkurve als auch die Daten anzeigt, wie Gesamtvolumen, Flowzeit und Anstiegszeit.

## Ansprüche

1. Einrichtung zur Messung der Harnströmung ( Uroflow) eines Patienten,aus einem mit einem Trichter und einem Ausflußstutzen versehenen Meßkopf und aus einer elektronischen Auswerteeinrichtung,
**dadurch gekennzeichnet**,
daß der Meßkopf (1) mit einem den Ausflußstutzen (30) bildenden geschlitzten Staurohr (4) und mit einem den Staudruck messenden Druckaufnehmer (18) versehen ist und daß die elektronische auswerteeinrichtung (9) die Flowwerte aus dem gemessenen flowproportionalen Staudruck errechnet.

2. Einrichtung nach Anspruch 1, dadurch gekennzeichnet, daß der Meßkopf (1) einen mit dem Trichter (2) verbundenen vertikalen Zulaufraum (12), einen vom geschlitzten Staurohr (4) durchdrungenen Abführraum (13), einen mit dem Druckaufnehmer (18) verbundenen vertikalen Druckmeßraum (16) und einen den Zulaufraum (12) ,den Abführraum (13) und den Druckmeßraum (16) verbindenden horizontalen Verteilraum (10) aufweist und daß die Unterkanten des vertikalen Schlitzes (14) im Staurohr (4), des Zulaufraumes (12) und des Druckmeßraumes (16) in einer gemeinsamen horzontalen Ebene (20) angeordnet sind, welche die obere Ebene des horizontalen Verteilraumes (10) bildet.

3. Einrichtung nach Anspruch 1 oder 2, dadurch gekennzeichnet, daß der Verteilraum (10) durch eine am Meßkopf (1) lösbar und flüssigkeitsdicht angeflanschte Schale (3) gebildet ist, in welcher das geschlitzte Staurohr (4) eingesetzt ist.

4. Einrichtung nach einem der Ansprüche 1 bis 3, dadurch gekennzeichnet, daß das Staurohr (4) einen vertikalen Schlitz (14) der Breite (5) aufweist .

5. Einrichtung nach einem der Ansprüche 1 bis 4, dadurch gekennzeichnet, daß der Meßkopf (1) mit dem Ausflußstutzen (30) auf einen Auffangbecher (6) aufgesetzt ist und daß mit einem weiteren, im Meßkopf (1) angeordneten Druckaufnehmer (22) das Gesamtvolumen des Urins durch Messung der Füllhöhe (H) des Auffangbehälters (6) über den statischen Druck des im Auffangbehälter gesammelten Urins gemessen wird.

6. Einrichtung nach einem der Ansprüche 1 bis 5, dadurch gekennzeichnet, daß beide Druckaufnehmer (18,22) in einem gemeinsamen Druckaufnehmergehäuse (8) angeordnet sind, das am Meßkopf (1) lösbar angeflanscht ist.

7. Einrichtung nach einem der Ansprüche 1 bis 6, dadurch gekennzeichnet, daß der zweite Druckaufnehmer (22) mit einem bis nahe dem Boden des Auffangbechers (6) geführten Meßrohr (5) versehen ist.

8. Einrichtung nach einem der Ansprüche 1 bis 7, dadurch gekennzeichnet, daß das Gesamtvolumen des Urins durch Messung der Füllhöhe (H) über den statischen Druck der im Auffangbehälter (6) gesammelten Flüssigkeit mittels des zweiten Druckaufnehmers (22) ermittelt wird und daß gleichzeitig mit dem ersten Druckaufnehmer (18) die flowproportionale Stauhöhe (h) am geschlitzten Staurohr (4) gemessen wird, wobei die Auswerte-Einrichtung (9) aus der flowproportionalen Stauhöhe (h) die Flow-Werte errechnet und gleichzeitig unter Zuhilfenahme der mit dem ersten Druckaufnehmer (18) ermittelten Volumenwerte die errechneten Flow-Werte korrigiert.

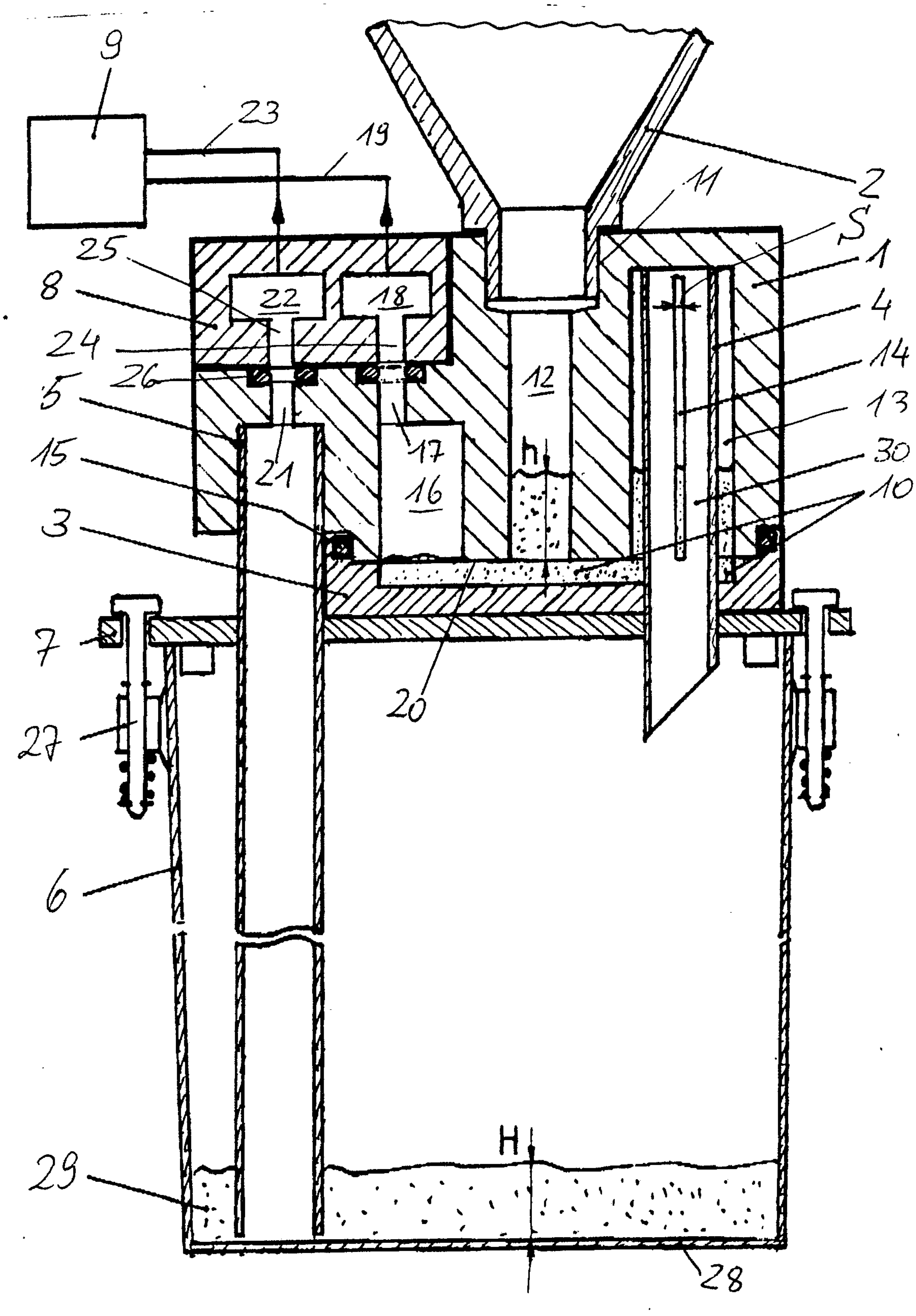
9
23
19
2
11
S
1
4
14
13
30
10
25
22
18
8
24
26
5
15
21
17
16
12
h
3
7
20
27
6
H
29
28

Europäisches Patentamt

# EUROPÄISCHER RECHERCHENBERICHT

Nummer der Anmeldung

EP 90 73 0011

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,A | US-A-4 554 687 (G.L. CARTER UND V.B. WEEKS) * Spalte 3, Zeile 6 - Spalte 6, Zeile 17 * * Spalte 7, Zeilen 45 - 57; Figuren * | 1,2,3 | A 61 B 5/20 G 01 F 1/52 |
| Y,A | US-A-4 100 802 (T.N. LAYTON) * Spalte 2, Zeile 33 - Spalte 3, Zeile 55 * * Spalte 6, Zeile 20 - Spalte 7, Zeile 9 @ Figuren * | 1,2,4 | |
| A | FR-A-2 362 369 (W. BECK ET R. THEDIECK) * Seite 10, Zeile 34 - Seite 15, Zeile 26 * * Figuren 7, 8 * | 1-3 | |
| A | US-A-4 589 280 (G.L. CARTER) * Spalte 4, Zeile 3 - Spalte 5, Zeile 40 * * Figuren * | 1,2 | |
| A | DE-A-2 623 557 (J. PÖRTENER) * Seite 5, Zeile 21 - Seite 7, Zeile 5 * * Seite 9, Zeile 15 - Seite 10, Zeile 9; Figuren * | 1,2,5,8 | |
| A | US-A-3 726 140 (G.G. BARBIE) * Spalte 2, Zeilen 13 - 55; Figuren * | 5,7,8 | |
| | | | RECHERCHIERTE SACHGEBIETE (Int. Cl.5) |
| | | | A 61 B G 01 F |

Der vorliegende Recherchenbericht wurde für alle Patentansprüche erstellt

| Recherchenort | Abschlußdatum der Recherche | Prüfer |
|---|---|---|
| Den Haag | 09 Januar 91 | RIEB K.D. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument